# EUROPÄISCHE PATENTANMELDUNG

(11) **EP 0 955 016 A2**
(43) Veröffentlichungstag der Anmeldung: **10.11.1999**
(21) Anmeldenummer: 99107941.9
(22) Anmeldetag: 22.04.1999
(51) Int. Cl.: A61F 2/06

(54) **Radioaktiver Stent**

(30) Priorität: 05.05.1998 DE 19819635
(71) Anmelder: Jomed Implantate GmbH, 72414 Rangendingen (DE)
(72) Erfinder: von Oepen, Dr.-Ing. Randolf, 72145 Hirrlingen (DE)
(74) Vertreter: Schmitz, Hans-Werner, Dipl.-Ing.

(57) **Zusammenfassung**

Stent zur Implantierung in ein Körpergefäß, der Rezeptoren zur Aufnahme von medizinischen Wirkstoffen aufweist.

## Beschreibung

Die Erfindung beschreibt einen Stent zur Implantierung in ein Körpergefäß.

Stents haben die Aufgabe, eine erneute Verengung bzw. einen erneuten Verschluss der Blutgefäße nach einer Dilatation zu verhindern, um einen guten Durchfluss des Blutes wieder sicherzustellen. Stents können jedoch nicht nur in Blutgefäßen, sondern auch in anderen Hohlgefäßen wie beispielsweise der Harnröhre oder den Gallengängen eingesetzt werden.

Zahlreiche Studien haben bewiesen, dass Stents die Neigung des Gefäßes, sich erneut zu verengen bzw. erneut zu verschließen, deutlich reduzieren können. Trotzdem kommt es beim Einsatz von Stents in ca. 30 % aller Fälle zu so genannten Restenosen, d. h. einer erneuten Verengung des Gefäßes im Bereich des Stents. Diese erneute Verengung kann beispielsweise durch eine Prolifikation der glatten Muskelzellen hervorgerufen werden.

Aus diesem Grund werden zahlreiche Bemühungen getätigt, diese Restenoserate zu reduzieren. Aus der Krebstherapie ist bekannt, dass eine Wucherung durch eine bestimmte Beaufschlagung der Zellen mit Radioaktivität reduziert bzw. unterdrückt werden kann. Die Zellen werden bei dieser Behandlung gezielt so geschädigt, dass ein weiteres Wachstum unterbunden wird.

In der US 5,722,984 C1 wird ein vorzugsweise metallischer Stent, welcher radioaktiv markiert ist, beschrieben. Nach der Einbringung in das erkrankte Gefäß soll die über der Zeit abklingende schwache Radioaktivität einer Wucherung der glatten Muskelzellen entgegenwirken.

Der Einsatz von radioaktiven Stents ist in der Praxis jedoch nur schwer möglich. So muss der Halbwertszeit der Produkte Rechnung getragen werden, welches zu großen logistischen Problemen führt. Radioaktive Stents können nur innerhalb eines sehr kurzen Zeitraums zum Einsatz kommen, da ansonsten eine genaue Dosierung der Radioaktivität nicht gesichert werden kann.

Große logistische Probleme sind auch beim Transport der Stents vom Hersteller zum Anwender zu erwarten. Je nach Auswahl der Strahlung und Höhe der Dosierung müssen für den Transport Sicherheitsmaßnahmen zur Abschirmung der Stents sichergestellt werden.

Abhängig von der Dosierung und der verwendeten Strahlung müssen in den einzelnen Katheterlabors ebenfalls umfangreiche Sicherheitsvorkehrungen getroffen werden. Zusätzlich kann eine solche Behandlung nur von geschulten Anwendern genutzt werden und ist somit der breiten Masse der anwendenden Kardiologen nicht zugänglich.

Der vorliegenden Erfindung liegt die Aufgabe zugrunde, einen Stent vorzuschlagen, der insbesondere Wucherungen von glatten Muskelzellen entgegenwirkt und dabei die oben genannten Nachteile vermeidet.

Die Aufgabe wird durch einen Stent mit den Merkmalen des Anspruchs 1 gelöst.

Die vorliegende Erfindung entkoppelt den Vorgang der Stentung und den Vorgang zur Einbringung der Radioaktivitat oder anderer medizinischer Wirkstoffe. Ein Stent wird nach bewährter Vorgehensweise vom Kardiologen in das erkrankte Gefäß über eine geeignete Vorrichtung, beispielsweise mittels einem Ballonkatheter, an der zu behandelnden Stelle platziert. Der Stent ist hierbei mit speziellen Rezeptoren ausgestattet, welche vorzugsweise eine hohe Affinität zu bestimmten Substanzen aufweisen. Diese Rezeptoren haften an der Oberfläche der Stents und erlauben die gezielte Ankopplung von Substanzen, die sich durch eine hohe Affinität zu den auf der Oberfläche des Stents befindlichen Rezeptoren auszeichnen.

Eine solche Substanz, ausgestattet mit einer hohen Affinität zu den Rezeptoren, kann beispielsweise mit Radioaktivität angereichert sein. Hiermit wird sichergestellt, dass ein Stent, ausgestattet mit den entsprechenden Rezeptoren, eine genau definierte Menge der in den Körper des Patienten, beispielsweise intravenös eingeleiteten Substanzen aufnehmen kann und somit eine genaue Dosis der Radioaktivität eingestellt werden kann.

Der Prozess der Bindung einer radioaktiven Substanz kann auch zwei- oder mehrstufig ablaufen. Der Stent verfügt wiederum durch gezielt auf der Oberfläche verankerten Rezeptoren über die Möglichkeit, spezielle Substanzen zu binden. Diese Substanz muss nicht primär eine Substanz sein, in welche die Radioaktivität eingekoppelt ist, sondern es kann sich auch um eine Substanz handeln, die ihrerseits eine große Affinität zu einer radioaktiven Substanz aufweist.

Die Entkoppelung des Prozesses zwischen Stentung und Aktivierung der medizinischen Wirksamkeit des Stents ermöglicht den behandelnden Kardiologen individuell zu entscheiden, ob eine solche Therapie gewünscht ist und welche Dosis für den entsprechenden Patienten zu wählen ist.

Die Aktivierung des Stents kann entweder direkt vom geschulten Personal im Katheterlabor durchgeführt werden, oder der Patient wird an Abteilungen überwiesen, die über die Technologie und die entsprechenden Sicherheitsmaßnahmen zur Strahlentherapie verfügen.

Wird der Stent mit unterschiedlichen Rezeptoren ausgestattet oder baut sich die angelagerte Substanz an den Rezeptoren über einen gewissen Zeitraum ab, so kann die Prozedur der Aktivierung gegebenenfalls wiederholt werden.

Die Rezeptoren können als Schicht auf den Stent aufgebracht werden. Weitere Ausführungsformen erfindungsgemäßer Stents können mit einer Hülle und/oder einer Auskleidung versehen sein. Die Auswahl des Stentmaterials ist nicht auf medizinischen Edelstahl begrenzt. Alle Materialien, die eine genügend hohe Akzeptanz im Organismus aufweisen, können Verwendung finden.

Es muss sichergestellt sein, dass sowohl die Rezeptoren als auch die in den Körper des Patienten eingeleiteten Substanzen eine ausreichende Körperverträglichkeit aufweisen.

Die Technologie des Aufbringens der Rezeptoren auf die Oberfläche von Stents ist keinesfalls auf die Zugabe von radioaktiven Substanzen begrenzt. Die Rezeptoren können auch so gestaltet werden, dass eine hohe Affinität zu Medikamenten besteht, welche beispielsweise dosiert über einen längeren Zeitraum in den Körper abgegeben werden sollen. Die Anlagerung solcher Medikamente an den Rezeptoren entspricht der Anlagerung radioaktiver Substanzen wie oben beschrieben.

Die Erfindung ist nicht auf die Ankopplung eines einzelnen Rezeptors beschränkt. Es können vielmehr unterschiedliche Rezeptoren auf der Oberfläche gebunden werden, um eine Behandlung mit unterschiedlichen Medikamenten durchzuführen.

Die Rezeptoren können auch im Material des Stents enthalten sein. Dies bietet sich insbesondere dann an, wenn eine rationelle Fertigung gewünscht wird. Um die medizinischen Wirkstoffe genauer dosieren zu können bzw. um eine überdosierung zu vermeiden, kann sich die Schicht und/oder die Hülle und/oder die Auskleidung nach einer bestimmten Zeit auflösen. Aus demselben Grund können die Rezeptoren auch so ausgebildet werden, dass sie ihre Affinität für gewisse Wirkstoffe nach einer bestimmten Zeit verlieren. Damit die Stents individuell je nach gewünschter Therapieform auch noch im Körper therapeutisch wirksam gemacht werden können, können die Rezeptoren mittels eines Katheters nachträglich aufgebracht werden.

## Patentansprüche

1. Stent zur Implantierung in ein Körpergefäß, dadurch gekennzeichnet, dass er Rezeptoren zur Aufnahme von medizinischen Wirkstoffen, insbesondere von radioaktiv markierten Wirkstoffen, aufweist.

2. Stent nach Anspruch 1, dadurch gekennzeichnet, dass die Rezeptoren eine hohe Affinität für radioaktiv markierte Wirkstoffe aufweisen.

3. Stent nach Anspruch 1 oder 2, dadurch gekennzeichnet, dass die Rezeptoren eine hohe Affinität für chemische Wirkstoffe aufweisen.

4. Stent nach einem der Ansprüche 1 bis 3, dadurch gekennzeichnet, dass er eine Schicht mit Rezeptoren für medizinische Wirkstoffe aufweist.

5. Stent nach einem der Ansprüche 1 bis 4, dadurch gekennzeichnet, dass er eine Hülle und/oder eine Auskleidung mit Rezeptoren für medizinische Wirkstoffe aufweist.

6. Stent nach einem der Ansprüche 4 oder 5, dadurch gekennzeichnet, dass sich die Schicht oder die Hülle und/oder die Auskleidung nach einer bestimmten Zeit auflöst.

7. Stent nach einem der Ansprüche 1 bis 6, dadurch gekennzeichnet, dass die Rezeptoren ihre Affinität für gewisse Wirkstoffe nach einer bestimmten Zeit verlieren.

8. Stent nach einem der Ansprüche 1 bis 7, dadurch gekennzeichnet, dass die Rezeptoren mittels eines Katheters nachträglich aufbringbar sind.

9. Stent nach einem der Ansprüche 1 bis 8, dadurch gekennzeichnet, dass die Rezeptoren im Material des Stents enthalten sind.

10. Stent nach einem der Ansprüche 1 bis 9, dadurch gekennzeichnet, dass die Wirkstoffe eine hohe Affinität für weitere Wirkstoffe, vorzugsweise für radioaktive Stoffe, aufweisen.

11. Stent nach einem der Ansprüche 1 bis 10, dadurch gekennzeichnet, dass die Wirkstoffe nach einer bestimmten Zeit vom Körper abgebaut werden.
